# EUROPEAN PATENT APPLICATION

(11) **EP 1 795 111 A1**
(43) Date of publication of application: **13.06.2007**
(21) Application number: 05781409.7
(22) Date of filing: 02.09.2005
(51) Int. Cl.: A61B 1/00

(54) **REMOVABLE FILTER UNIT AND ENDOSCOPE**

(30) Priority: 07.09.2004 JP 2004259442
(71) Applicant: Olympus Corporation, Tokyo 151-0072 (JP)
(72) Inventor: XIE, Tianyu, 1970821 (JP)
(74) Representative: von Hellfeld, Axel
(86) International application number: PCT/JP2005/016144
(87) International publication number: WO 2006/028023

(57) **Abstract**

A removable filter apparatus and an endoscope using the same which can easily select and transmit fluorescences of different wavelengths are provided. There is provided a removable filter apparatus 1 which is attached, in an attachable and removable manner, to a tip 32 of an inserting portion 30 of an endoscope apparatus including a projection unit 33 for emitting light towards biological tissue and a solid-state image-acquisition device 35 for detecting return light from the biological tissue, the removable filter apparatus 1 comprising a plurality of filters 50a and 50b for transmitting fluorescences of different wavelengths among the return light from the biological tissue and for blocking excitation light which generates the fluorescences.

## Description

### Technical Field

The present invention relates to a removable filter apparatus of a video endoscope apparatus for observing fluorescences of a plurality of wavelengths and to an endoscope apparatus using the same.

### Background Art

In the related art, there are known techniques for observing autofluorescence from an organism or fluorescence of a substance injected into an organism to diagnose diseases in biological tissue, such as degeneration and cancer.

For example, if the biological tissue is irradiated with excitation light, fluorescence with a longer wavelength than the excitation light is generated. Substances which produce fluorescence in response to excitation light are also present inside organisms, and it is becoming clear that there are relationships between those substances and diseases. It is also known that there are fluorescers having fluorescent pigments which attach to cancer cells. Because more of these fluorescers than normal cells gather in cancer cells, by injecting these fluorescers into an organism, it is possible to diagnose cancerous sites.

Methods for carrying out fluorescence observation using endoscopes, as described above, have also been proposed, and it is possible to diagnose cancer and so on inside body cavities using endoscopes.

Examples of such endoscopes include videoscopes for guiding light to the exterior using an optical fiber and observing it, and videoscopes which observe light using a CCD device provided at the tip portion of the endoscope; both of these are used to carry out diagnosis of cancer and so on inside body cavities. Fiberscopes have the advantage that their diameter can be made smaller than that of videoscopes, and videoscopes have the advantage that their image resolution can be higher than that of fiberscopes.

For videoscopes, many technologies have been proposed concerning methods of arranging and constructing them in order to provide CCD devices, optical filters, and so forth at the tip portions thereof (for example, see Patent Documents 1 and 2).
Patent Document 1: Japanese Unexamined Patent Application Publication No. HEI-8-140928
Patent Document 2: Japanese Unexamined Patent Application Publication No. HEI-8-224208

### Disclosure of Invention

Patent Document 1 described above discloses an endoscope provided with a CCD device for detecting visible light and a CCD device for detecting fluorescence of one predetermined wavelength.

In this endoscope for detecting one wavelength, when a fluorescer that produces fluorescence in cancer cells, for example, is administered to the patient, it cannot be determined to which sites the fluorescer spreads. Therefore, there is a problem in that it is not possible to determine whether no fluorescence is detected because cancer cells do not exist, or whether no fluorescence is detected because the fluorescer has not spread; therefore, it is not possible to easily detect cancer cells and so on.

In order to overcome this problem, Patent Document 2 described above discloses an endoscope which can detect visible light and which can also detect fluorescences of two different wavelengths.

Therefore, in the endoscope which can detect fluorescences of two different wavelengths, for example, by administering to the patient one fluorescer which produces fluorescence of a predetermined wavelength in cancer cells and another fluorescer which produces fluorescence of a different wavelength from the predetermined wavelength, it is possible to detect to which sites the two fluorescers spread. In other words, because the degrees of spreading of both fluorescers are substantially the same, by detecting the fluorescence emitted from the other fluorescer, it is possible to detect to which sites the first fluorescer has spread.

As a result, it is possible to decide whether no fluorescence is detected because cancer cells do not exist, or whether no fluorescence is detected because the fluorescer has not spread. Therefore, it is easy to detect cancer cells and so on.

However, in Patent Documents 1 and 2 described above, in order to excite fluorescence from either fluorescer, the fluorescer is radiated with excitation light having a higher intensity (brighter) than the fluorescence. In addition, a filter for blocking the excitation light and transmitting the fluorescence is disposed at the light-incident side of a CCD device or the like, and the fluorescence is detected.

In other words, it is necessary to match the characteristics of the fluorescer and the characteristics of the endoscope, that is to say, the wavelengths of the excitation light and the fluorescence according to the fluorescer, with the transmission and blocking characteristics of the filter provided in the endoscope. Therefore, it is necessary to employ endoscopes according to, for example, the number of fluorescers used in the cancer-cell detection, which causes a problem in that it is not possible to easily detect cancer cells and so on.

Moreover, it is difficult to construct filters corresponding to numerous fluorescers, that is, filters for blocking numerous excitation light beams and for transmitting a plurality of fluorescences, because doing so would be expensive. Therefore, there is a problem in that it is not possible to easily detect cancer cells and so on.

The present invention has been conceived to solve the problems described above, and an object thereof is to provide a removable filter apparatus and an endoscope apparatus using the same, which can easily select and transmit fluorescences of different wavelengths.

In order to realize the objects described above, the present invention provides the following solutions.

According to Claim 1, the invention provides a removable filter apparatus which is attached, in an attachable and removable manner, to a tip of an inserting portion of an endoscope apparatus including a projection unit for emitting light towards biological tissue and a solid-state image-acquisition device for detecting return light from the biological tissue, the removable filter apparatus comprising a plurality of filters corresponding to fluorescences of different wavelengths in the return light from the biological tissue, wherein the plurality of filters block light with wavelengths shorter than the wavelengths of the corresponding fluorescences.

According to the present invention, because it is possible to replace the plurality of filters by replacing the removable filter apparatus, the wavelengths of the light blocked by the removable filter apparatus, for example, excitation light and transmitted fluorescence, can be easily selected and changed.

Therefore, by selecting and replacing the removable filter apparatus according to the wavelengths of, for example, the fluorescence to be observed and the excitation light for generating the fluorescence, it is possible to observe fluorescences of different wavelengths with a single endoscope.

In addition, because the removable filter apparatus has a plurality of filters, it is possible to simultaneously block light with wavelengths longer than the wavelengths of the corresponding fluorescences and to transmits a plurality of fluorescences.

Therefore, when observing biological tissue to which a plurality of different fluorescers have been administered, for example, it is possible to simultaneously observe the fluorescences produced by the individual fluorescers using a removable filter apparatus having filters corresponding to the individual fluorescers.

In the invention described above, the plurality of filters preferably block light with wavelengths longer than the wavelengths of the corresponding fluorescences.

According to the present invention, the plurality of filters further block light with wavelengths longer than the wavelengths of the corresponding fluorescences. Therefore, using a plurality of filters, it is possible to observe only the corresponding fluorescences.

In the invention described above, the plurality of filters are preferably disposed at positions which cover parts of light-receiving surfaces of the corresponding solid-state image-acquisition devices; and a branching unit for branching the return light from the biological tissue to the plurality of filters is provided.

According to the present invention, because the branched light beams branched by the branching unit are made incident on different corresponding parts of the light-receiving surface of the solid-state image-acquisition device, it is possible to simultaneously acquire a plurality of images of the biological tissue with a single solid-state image-acquisition device.

Furthermore, because the split light beams are made incident on different filters and the fluorescences transmitted through the filters are made incident on the light-receiving surface of the solid-state image-acquisition device, it is possible to simultaneously acquire a plurality of fluorescence images from the biological tissue using a single solid-state image-acquisition device.

In the invention describe above, the branching unit is preferably a polarizer.

According to the present invention, for example, the light is divided by the polarizer into linear polarizations whose oscillation directions are mutually orthogonal. Images represented by the divided linear polarizations, as well as being the same as each other, are also the same as the image represented by the light incident on the polarizer. Therefore, it is possible to simultaneously acquire a plurality of fluorescence images issuing from the same region of the biological tissue with a single solid-state image-acquisition device.

By using two or more polarizers, it is possible to divide the light into three or more parts.

In the invention described above, the branching unit is preferably a splitter.

According to the present invention, the light is divided into a plurality of branched beams by the splitter. The images represented by the branched beams, as well as all being the same, are also the same as the image represented by the light incident on the splitter. Therefore, it is possible to simultaneously acquire a plurality of fluorescence images issuing from the same region of the biological tissue with a single solid-state image-acquisition device.

As the splitter, it is preferable to use a component including a mirror which reflects part of the incident light and transmits the rest.

In the invention described above, preferably, two of the solid-state image-acquisition devices are provided; and at least one of the filters is disposed at a position which covers the light-receiving surfaces of the respective solid-state image-acquisition devices.

According to the present invention, it is possible to make the fluorescence which is transmitted through the filter incident on the light receiving surfaces of both solid-state image-acquisition devices. Therefore, both solid-state image-acquisition devices can detect the fluorescence from the biological tissue.

In the invention described above, of the plurality of filters, at least one of the filters preferably transmits visible light.

According to the present invention, it is possible to observe a visible-light image of the biological tissue with the solid-state image-acquisition device. Therefore, it is possible, for example, to simultaneously observe and detect a visible-light image and a fluorescence image, and by using both images, the site which produces the fluorescence can be more accurately identified.

The invention described above preferably further comprises an elongated light guide for guiding the light emitted from the projection unit to an end surface.

According to the present invention, it is possible to emit the light from the elongated light guide towards the biological tissue. Therefore, even though the removable filter is attached to the tip of the inserting portion, the light emitted from the projection unit is not blocked.

In the invention described above, an elongated treatment-instrument channel, which extends a treatment-instrument channel provided in the endoscope apparatus up to the end surface, is preferably formed.

According to the present invention, it is possible to guide a treatment instrument towards the biological tissue by means of the elongated treatment-instrument channel. Therefore, even though the removable filter is attached to the tip of the inserting portion, the treatment instrument which is guided via the treatment-instrument channel is not blocked.

The invention according to Claim 9 provides an endoscope apparatus comprising a light source for emitting light; a switching mechanism for converting the light emitted from the light source into light of a plurality of different wavelengths; a light guide, disposed inside an inserting portion for inserting into a body cavity, for guiding the light converted by the switching mechanism to a tip of the inserting portion and for emitting the light towards biological tissue; a solid-state image-acquisition device, disposed at the tip of the inserting portion, for detecting return light from the biological tissue; and a removable filter apparatus which is attached, in an attachable and removable manner, to the tip of the inserting portion, and which includes a plurality of filters for transmitting fluorescences of different wavelengths among the return light from the biological tissue and for blocking excitation light which generates the fluorescences.

According to the present invention, by replacing the removable filter apparatus according to, for example, the wavelengths of the fluorescences to be observed and the excitation light for exciting the fluorescences, it is possible to observe fluorescences of different wavelengths with a single endoscope.

In addition, because the removable filter apparatus has a plurality of filters, when observing, for example, biological tissue to which a plurality of different fluorescers have been administered, it is possible to simultaneously observe the fluorescences issuing from the individual fluorescers.

According to the removable filter apparatus of the present invention, it is possible to replace the removable filter apparatus according the wavelengths of the fluorescences transmitted. Therefore, an advantage is provided in that it is possible easily select and transmit fluorescences of different wavelengths. Also, the removable filter apparatus has a plurality of filters which block and transmit excitation light and fluorescences of different wavelengths. Therefore, an advantage is provided in that it is possible to simplify selection of the fluorescences with different wavelengths and to transmit simultaneously transmit them.

According to the endoscope apparatus of the present invention, it is possible to replace the removable filter apparatus according to the wavelengths of the fluorescences to be observed. Therefore, an advantage is provided in that it is possible to easily observe fluorescences of different wavelengths with a single endoscope apparatus. In addition, an advantage is provided in that it is possible to easily perform simultaneous observation of fluorescences having different wavelengths.

### Brief Description of Drawings

[FIG. 1] Fig. 1 is an outline configuration diagram showing an endoscope apparatus according to a first embodiment of the present invention.
[FIG. 2] Fig. 2 is a plan view of a filter turret shown in Fig. 1.
[FIG. 3] Fig. 3 are diagrams showing the wavelength distribution of light emitted from a light source shown in Fig. 1 and a light transmittance characteristic of each filter in the filter turret shown in Fig. 2.
[FIG. 4] Fig. 4 is an enlarged cross-section of a tip portion of an endoscope shown in Fig. 1.
[FIG. 5] Fig. 5 are diagrams showing the wavelength distribution of light emitted from the light source unit shown in Fig. 1 and the light transmittance characteristic of each filter in a filter portion shown in Fig. 4.
[FIG. 6] Fig. 6 is a diagram showing an example of a fluorescence image of a lesion displayed on a monitor shown in Fig. 1.
[FIG. 7] Fig. 7 is an enlarged cross-section of a tip portion of an endoscope in an endoscope apparatus according to a second embodiment of the present invention.
[FIG. 8] Fig. 8 is a diagram showing an example of a visible-light image and a fluorescence image of a lesion displayed on the monitor shown in Fig. 1.
[FIG. 9] Fig. 9 is an enlarged cross-section of a tip portion of an endoscope in an endoscope apparatus according to a third embodiment of the present invention.
[FIG. 10] Fig. 10 is an enlarged cross-section of a tip of an endoscope in an endoscope apparatus according to another example of the third embodiment of the present invention.
[FIG. 11] Fig. 11 is an enlarged cross-section of a tip of an endoscope in an endoscope apparatus according to a fourth embodiment of the present invention.
[FIG. 12] Fig. 12 are diagrams showing the wavelength distribution of light emitted from the light source unit shown in Fig. 1 and a light transmittance characteristic of each filter in a filter section shown in Fig. 11.

### Best Mode for Carrying Out the Invention

### First Embodiment

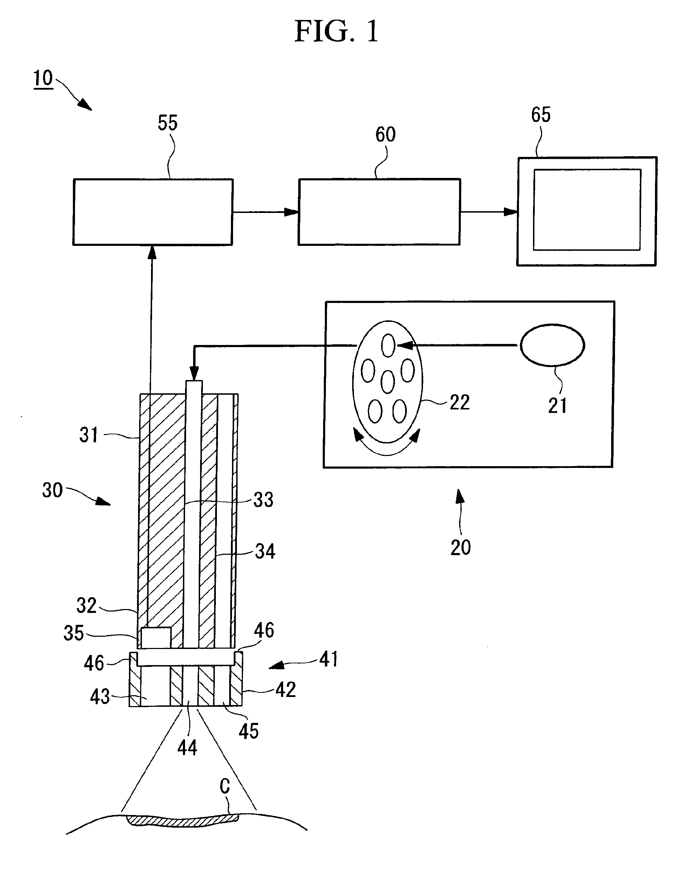

A first embodiment of the present invention will be described below with reference to Figs. 1 to 6.

Fig. 1 is a diagram showing the overall configuration of an endoscope apparatus according to a first embodiment of the present invention for diagnosing lesions using fluorescence.

As shown in Fig. 1, the endoscope apparatus 10 is principally formed of a light source unit (projection unit) 20 for emitting visible light, excitation light, and so on; an endoscope (inserting portion) 30 for insertion into the body cavity of a living organism to form an image of a lesion (biological tissue) C; a camera control unit 55 for converting the image signal formed by the endoscope 30 into a video signal; an image processor 60 for processing the video signal to make it easier to recognize the lesion C and a normal region; and a monitor 65 for displaying the output from the image processor 60.

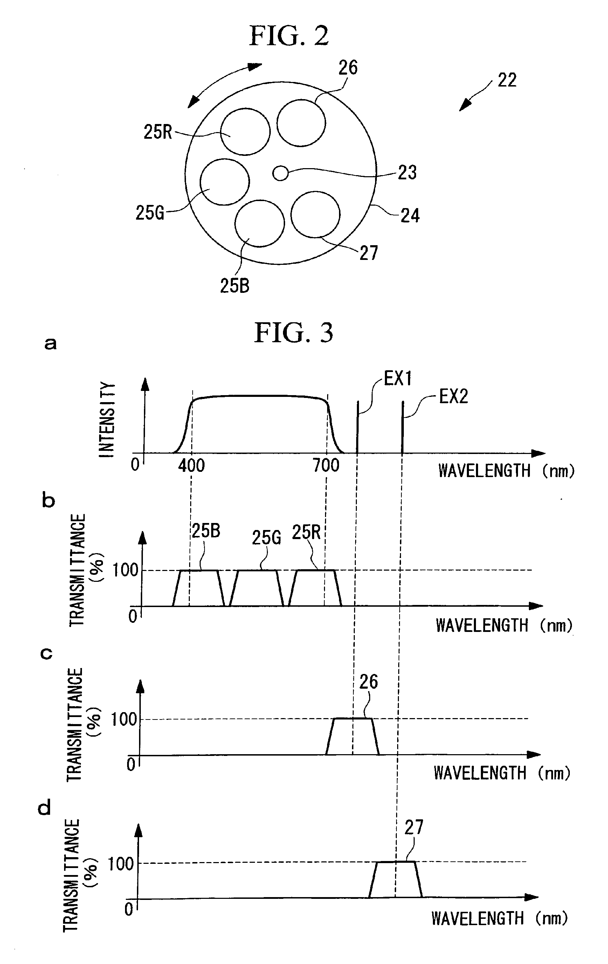

Fig. 2 is a plan view of a filter turret 22 provided in the light source unit 20 of the endoscope apparatus 10.

As shown in Figs. 1 and 2, the light source unit 20 is formed of a light source 21, for example, a xenon lamp, and the filter turret (switching mechanism) 22 for converting the light emitted from the light source 21. The filter turret 22 is formed of a circular plate 24 which rotates about a rotation shaft 23; a blue-light filter 25B for transmitting blue light (visible light) B; a green-light filter 25G for transmitting green light (visible light) G; a red filter 25R for transmitting red light (visible light) R; a first excitation-light filter 26 for transmitting first excitation light (excitation light) EX1; and a second excitation-light filter 27 for transmitting second excitation light (excitation light) EX2. Each of the filters 25B, 25G, 25R, 26, and 27 is disposed on substantially the same circumference of the circular plate 24, and the filter turret 22 is disposed so the light from the light source 21 is incident on one of the filters 25B, 25G, 25R, 26, and 27.

Fig. 3(a) is a diagram showing the wavelength distribution of the light emitted from the light source unit 20; Fig. 3(b) is a diagram showing transmittance characteristics versus light wavelength for the blue-light filter 25B, the green-light filter 25G, and the red-light filter 25R; Fig. 3(c) is a diagram showing the transmittance characteristics with respect to light of the first excitation-light filter; and Fig. 3(d) is a diagram showing the transmission characteristics with respect to light of the second excitation-light filter.

As shown in Fig. 3(a), the light emitted from the light source 21 includes all light of each of the wavelengths B, G, R, EX1, and EX2. Therefore, when the light emitted from the light source 21 is radiated onto the blue-light filter 25B, the green-light filter 25G, and the red-light filter 25R, as shown in Fig. 3(b), B, G, and R light is respectively transmitted. When the light emitted from the light source 21 is radiated onto the first excitation-light filter 26 and the second excitation-light filter 27, as shown in Figs. 3(c) and (d), EX1 and EX2 are respectively transmitted.

As shown in Figs. 1 and 2, because the light from the light source 21 is time-sequentially radiated onto each filter 25B, 25G, 25R, 26, and 27 by rotating the circular plate 24, each of the B, G, R, EX1, and EX2 light beams is time-sequentially emitted from the light source unit 20.

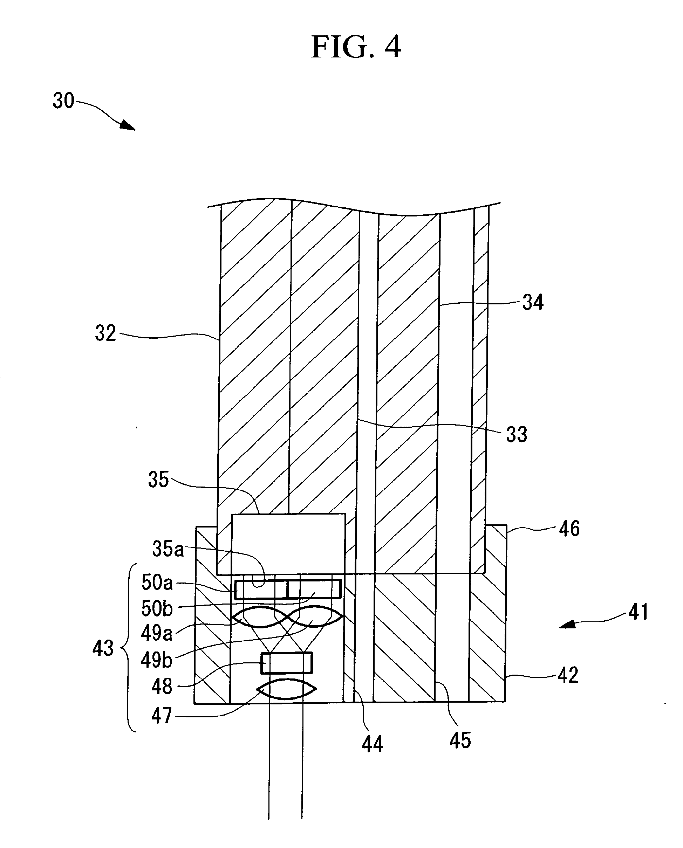

Fig. 4 is an enlarged cross-sectional view of the tip portion of the endoscope 30, which is inserted into the body cavity.

As shown in Figs. 1 and 4, the endoscope 30 is mainly formed of a scope main body 31 and a cap (removable filter apparatus) 41 which is attached to the tip portion (tip) 32 thereof in such a manner that it can be attached thereto and removed therefrom.

A light guide (projection unit) 33 for guiding each of the B, G, R, EX1, and EX2 light beams emitted from the light source 20 into the body cavity and an endoscope channel (treatment-instrument channel) 34 for guiding a treatment instrument, such as forceps, into the body cavity are provided in the scope main body 31. An image-acquisition device (solid-state image-acquisition device) 35, such as a CCD device, for forming a fluorescence image of the lesion C is provided in the tip portion 32.

As shown in Fig. 4, the cap 41 is principally formed of a main body 42, a filter portion 43 for selectively transmitting fluorescence from the lesion C, an elongated light guide 44 for guiding the light from the light guide 33 to the tip of the cap 41; and an elongated endoscope channel (elongated treatment-instrument channel) 45 for guiding a treatment instrument to the tip of the cap 41. When the cap 41 is attached to the tip portion 32, the filter portion 43, the elongated light guide 44, and the elongated endoscope channel 45 are disposed opposite the image-acquisition device 35, the light guide 33, and the endoscope channel 34, respectively.

An attaching portion 46 for attaching to the tip portion 32 is disposed on the main body 42. The shape of the attaching portion 46 should allow the cap 41 to be attached to and removed from the tip portion 32, and any known method can be used to attach it.

The filter portion 43 is formed of a lens 47 for guiding the fluorescence from the lesion C to a polarizer (branching unit) 48; the polarizer 48 for dividing incident light into p-polarized light and s-polarized light; a first lens 49a and a second lens 49b for focusing the divided p-polarized light and s-polarized light onto the image-acquisition device 35; and a first filter (filter) 50a and a second filter (filter) 50b for transmitting fluorescence of respective predetermined wavelengths.

The polarizer 48 is disposed so that it divides the fluorescence which is incident from the lens 47 into p-polarized light and s-polarized light, and they are respectively made incident on the first lens 49a and the second lens 49b. As the polarizer 48, it is possible to use, for example, a Wollaston prism in which two uniaxial crystals are combined such that their optical axis are perpendicular.

The first lens 49a and the second lens 49b are disposed such that the p-polarized light and the s-polarized light divided by the polarizer 48 are respectively incident thereon, and such that each polarization focused by the first lens 49a and the second lens are imaged at the light-receiving surface 35a of the image-acquisition device 35. The first lens 49a and the second lens 49b are disposed such that the images of each polarization are formed in different halves of the light-receiving surface 35a.

As shown in Fig. 4, the first filter 50a is disposed between the first lens 49a and the image-acquisition device 35; and the second filter 50b is disposed between the second lens 49b and the image-acquisition device 35.

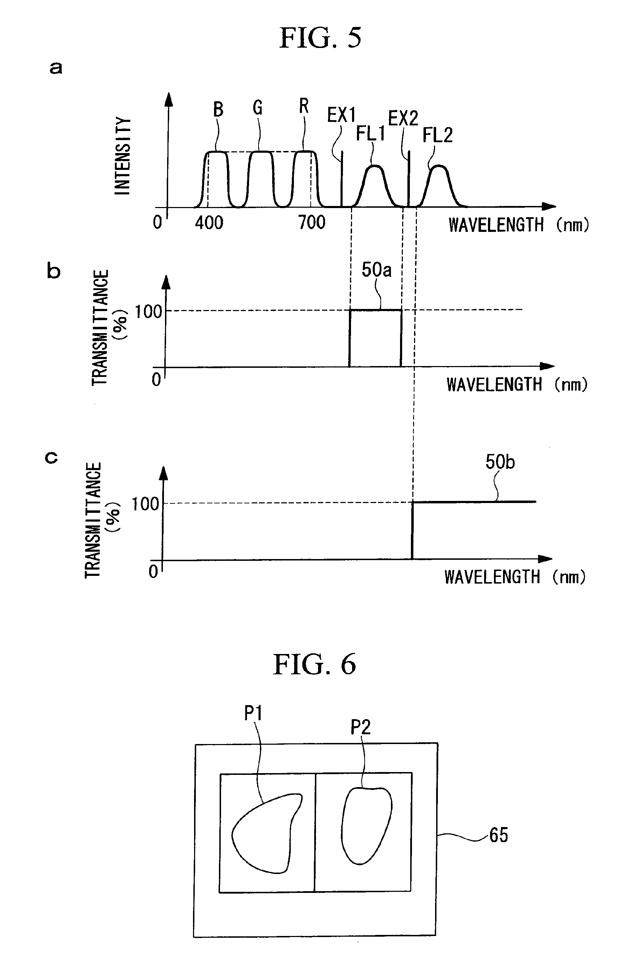

Fig. 5(a) is a diagram showing the relationships of the wavelengths of the B, G, R, EX1, and EX2 light and the wavelengths of first fluorescence FL1 excited by the first excitation light EX1 and second fluorescence FL2 excited by the second excitation light EX2. Fig. 5(b) is a diagram showing the transmittance characteristic with respect to incident light wavelength of the first filter 50a; and Fig. 5(c) is a diagram showing the transmittance characteristic with respect to incident light wavelength of the second filter 50b.

As shown in Fig. 5(b), a filter exhibiting a transmission characteristic with respect to the first fluorescence (fluorescence) FL1 excited by the first excitation light EX1 and exhibiting a light-blocking characteristic with respect to light of other wavelengths, especially the first excitation light EX1, is used as the first filter 50a. As shown in Fig. 5(c), a filter exhibiting a transmission characteristic with respect to the second fluorescence (fluorescence) FL2 excited by the second excitation light EX2 and exhibiting a light-blocking characteristic with respect to light of wavelengths shorter than that of the second fluorescence FL2 is used as the second filter 50b.

Next, the operation of the endoscope apparatus 10 having the above-described configuration will be described.

When electrical power is supplied to the light source 21, as shown in Figs. 1 and 2, light (illumination light) including all wavelengths from the blue light B to the second excitation light EX2 is emitted from the light source 21 towards the filter turret 22. The filter turret 22 rotates about the rotation shaft 23 and the illumination light irradiates each of the rotating filters 25B, 25G, 25R, 26, and 27.

The B, G, R, EX1, and EX2 light beams corresponding to the filters 25B, 25G, 25R, 26, and 27 are emitted in turn (time-sequentially) from the filter turret 22 according to the rotational period of the filter turret 22, and the B, G, R, EX1, and EX2 light beams are incident on the light guide 33 of the endoscope 30. The B, G, R, EX1, and EX2 light beams, which are guided into the body cavity by the light guide 33, are further guided in the elongated light guide 44, are emitted into the body cavity, and illuminate the lesion C.

A first fluorescer which produces the first fluorescence FL1 in response to the first excitation light EX1 and a second fluorescer which attaches to cells (for example, cancer cells) involved in the pathology of the lesion C and which produces second fluorescence FL2 in response to the second excitation light EX2 are administered to the lesion C in advance.

Accordingly, the first fluorescence FL1 is emitted from the region in the lesion C where the first fluorescer penetrates, and the second fluorescence FL2 is emitted from the cancer cells.

As shown in Fig. 4, light beams B, G, R, EX1, and EX2 and the fluorescences FL1 and FL2 are incident on the polarizer 48 via the lens 47 and are split into p-polarized light and s-polarized light. The p-polarized light and s-polarized light are incident on the first filter 50a and the second filter 50b, respectively. As shown in Fig. 5, only the first fluorescence FL1 is transmitted through the first filter 50a, and light of other wavelengths is blocked. Only the second fluorescence F12 is transmitted through the second filter 50b, and light of other wavelengths is blocked.

As shown in Fig. 4, the first fluorescence FL1 transmitted through the first filter 50a is incident on the left half of the light-receiving surface 35a of the image-acquisition device 35, and an image is formed by the first fluorescence FL1. The second fluorescence FL2 transmitted through the second filter 50b is incident on the right half of the light-receiving surface 35a, and an image is formed by the second fluorescence FL2.

As shown in Fig. 1, the image-acquisition device 35 converts the images of the fluorescence FL1 and FL2 formed at the light-receiving surface 35a into electrical signals and outputs them to the camera control unit 55. The electrical signals are converted to video signal in the camera control unit 55 and are output to the image processor 60. The video signals are subjected to signal processing in the image processor 60 to make it easy to recognize the lesion C and normal regions and are output to the monitor 65.

As shown in Fig. 6, an image P1 of the first fluorescence and an image P2 of the second fluorescence, which are subjected to signal processing, are displayed side-by-side on the monitor 65.

According to the configuration described above, by replacing the cap 41, it is possible to easily replace the first filter 50a and the second filter 50b. Therefore, it is possible to easily change the wavelength of light which is transmitted through the filter unit 43 and incident on the image-acquisition device 35.

For example, by selecting the cap 41 according to the wavelength of the fluorescence to be observed, it is possible to observe fluorescence having a plurality of different wavelengths using a single endoscope apparatus 10. More specifically, because the type of fluorescer used changes according to the type of lesion C, the wavelength of the excitation light for exciting the fluorescer and the wavelength of the fluorescence change. By preparing caps 41 according to these optical wavelengths and exchanging them, it is possible to observe fluorescence having a plurality of different wavelengths using a single endoscope apparatus 10.

Compared to a case where the entire endoscope apparatus 10 is replaced, by replacing only the cap 41, which is extremely low cost, it is possible to support a plurality of types of fluorescers and it is possible to perform examinations at even lower cost.

Using the polarizer 48, it is possible to split the light incident on the filter section 43 into two, the p-polarized light and the s-polarized light, without compromising the image of that light (the intensity of the light becomes substantially half). Also, because the p-polarized light and the s-polarized light are incident on corresponding halves of the light-receiving surface 35a of the image-acquisition device 35, it is possible to reduce the diameter of the tip 32 of the endoscope 31.

Accordingly, the endoscope 10 can observe images of the two fluorescences FL1 and FL2 at the same time, and the diameter of the tip 32 can be reduced.

Because the endoscope 10 can observe images of the two fluorescences FL1 and FL2, it is possible to observe the lesion C into which fluorescers with two different characteristics have been administered.

For example, as one fluorescer, it is possible to use a fluorescer which attaches to the cells involved in the pathology of the lesion C and which emits fluorescence in response to the excitation light, and as the other fluorescer, it is possible to use a fluorescer which emits fluorescence of a different wavelength in response to the excitation light. In such a case, it is possible to observe the degree of spreading of both administered fluorescers using the other fluorescer, and it is possible to observe the presence or absence of cells involved in the disease using the one fluorescer.

The elongated light guide 44 can emit the light beams B, G, R, EX1, and EX2 guided by the light guide 33 towards the lesion C without interference by the cap 41. Therefore, even if the cap 41 is attached to the tip 32 of the endoscope 31, it does not block the light beams B, G, R, EX1, and EX2 emitted from the light source unit 20.

The elongated channel 45 can guide a treatment instrument, which is guided by the endoscope channel 34, to the lesion C without interference by the cap 41. Therefore, even if the cap 41 is attached to the tip 32 of the endoscope 31, it is possible to guide the treatment instrument to the lesion C.

As described above, using the filter which is provided in the cap 41 and which transmits fluorescence of different wavelengths, it is possible to observe different fluorescence images, and by replacing the cap 41, using a filter which transmits fluorescence and a filter which transmits visible light (light composed of blue light B, green light G, red light R), it is possible to observe a fluorescence image and a visible-light image.

By providing two or more polarizers 48, it is possible to split the light into three or more beams. By doing so, it is possible to simultaneously acquire an even greater number of fluorescence images.

### Second Embodiment

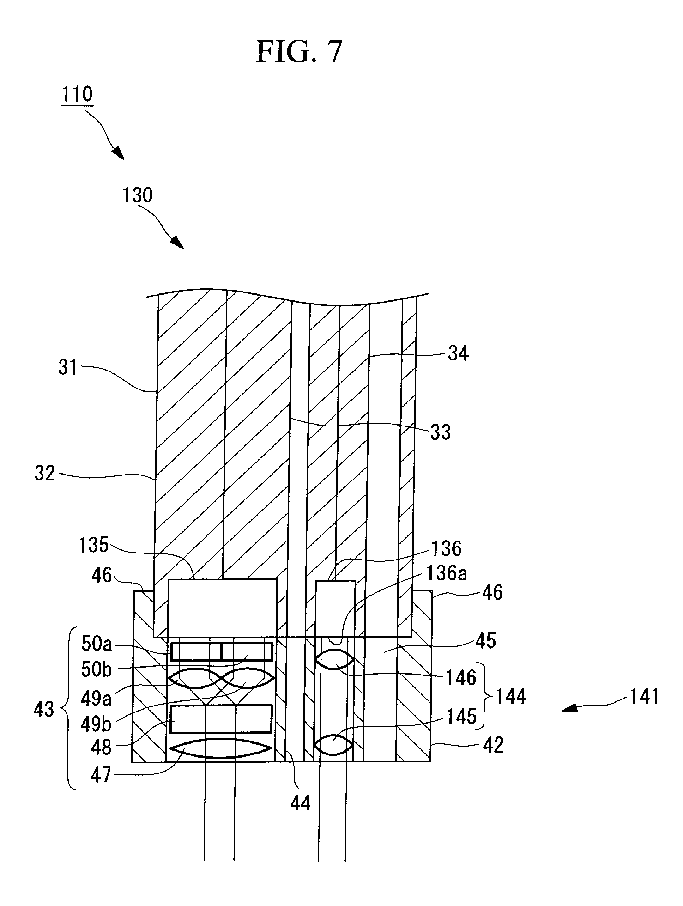

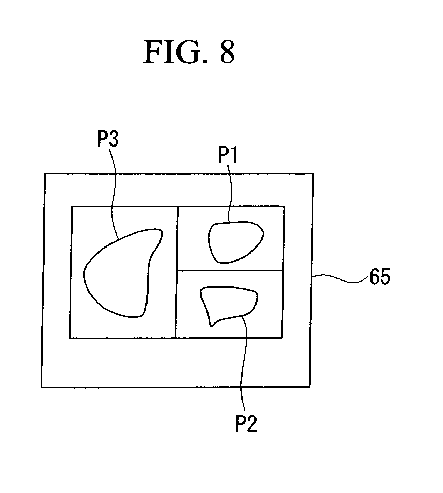

Next, a second embodiment of the present invention will be described with reference to Figs. 7 and 8.

The basic configuration of the endoscope apparatus of this embodiment is the same as that of the first embodiment, but the configuration of the endoscope is different from that of the first embodiment. Therefore, only the vicinity of the endoscope in this embodiment will be described using Figs. 7 and 8, and a description of the light source apparatus and so on will be omitted.

Fig. 7 is an enlarged cross-sectional view of an tip portion, which is inserted into a body cavity, of the endoscope apparatus according to this embodiment.

Elements which are identical to those of the first embodiment are assigned the same reference numerals, and a description thereof shall be omitted.

As shown in Fig. 7, an endoscope (inserting portion) 130 of the endoscope apparatus 110 is mainly formed of a scope main body 31 and a cap (removable filter apparatus) 141 which is attached to a tip portion 32 thereof in such a manner that it can be attached thereto and removed therefrom.

A first image-acquisition device (solid-state image-acquisition device) 135, such as a CCD device or the like, for acquiring a fluorescence image of the lesion C and a second image-acquisition device (solid-state image-acquisition device) 136 for acquiring a visible-light image of the lesion C are provided in the tip portion 32.

The cap 141 is mainly formed of a main body 42, a filter portion 43, an elongated light guide 44, an elongated endoscope channel 45, and a light-guiding portion 144 for guiding visible light from the lesion C to the second image-acquisition device 136. When the cap 141 is attached to the tip portion 32, the filter portion 43, the light-guiding portion 144, the elongated light guide 44, and the elongated endoscope channel 45 are disposed opposite the first image-acquisition device 135, the second image-acquisition device 136, a light guide 33, and an endoscope channel 34, respectively.

The light-guiding portion 144 is formed of a first light-guiding lens 145 and a second light-guiding lens 146 which form a visible-light image of the lesion C at a light-receiving surface 136a of the second image-acquisition device 136.

Next, the operation of the endoscope apparatus 110 having the above-described configuration will be described.

As shown in Fig. 7, in the endoscope apparatus 110, the images of the fluorescences FL1 and FL2 from the lesion C are converted to an electrical signal by the first image-acquisition device 135 and are output to the camera control unit described above.

Also, the visible light from the lesion C is focused onto the light-receiving surface 136a of the second image-acquisition device 136 by the first light-guiding lens 145 and the second light-guiding lens 146, and a visible-light image is formed. The visible-light image is converted to an electrical signal by the second image-acquisition device 136 and is output to the camera control unit described above.

Similarly to the first embodiment, after the acquired images of each light beam are converted to video signals in the camera control unit and are subjected to signal processing in the image processor, they are output to the monitor.

As shown in Fig. 8, a visible-light image P3 of the lesion C is displayed on the left half of the screen, and a first fluorescence image P1 and a second fluorescence image P2, which are subjected to signal processing, are displayed one above another on the right half.

According to the configuration described above, it is possible to observe a visible-light image of the lesion C using the second image-acquisition device 136. Therefore, it is possible to simultaneously observe a visible-light image P3 and fluorescence images P1 and P2, and by using these images, it is possible to more accurately identify the location of cells involved in the pathology.

### Third Embodiment

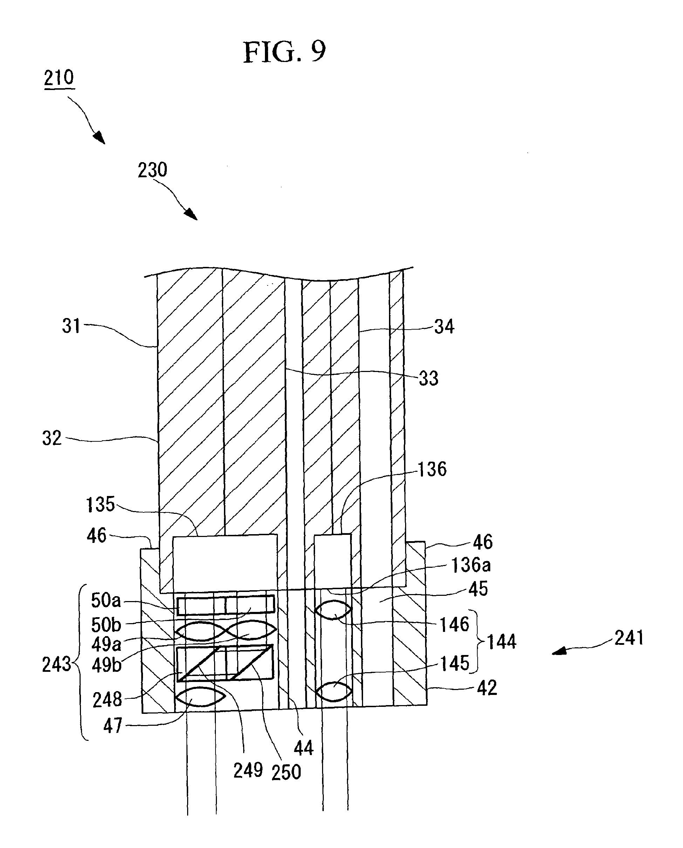

Next, a third embodiment of the present invention will be described with reference to Fig. 9.

The basic configuration of the endoscope apparatus according to this embodiment is the same as that of the second embodiment, but the configuration of the filter portion differs from that of the second embodiment. Therefore, in this embodiment, only the vicinity of the filter portion will be described using Fig. 9, and a description of the light source apparatus and so on will be omitted.

Fig. 9 is an enlarged cross-sectional view of the tip portion, which is inserted into the body cavity, of the endoscope apparatus according to this embodiment.

The same reference numerals are assigned to elements which are identical to those of the second embodiment, and a description thereof shall be omitted.

As shown in Fig. 9, the endoscope 230 of the endoscope apparatus 210 mainly includes a scope main body 31 and a cap (removable filter apparatus) 241 which is attached to a tip portion 32 thereof in such a manner that it can be attached thereto and removed therefrom.

A first image-acquisition device 135, such as a CCD device or the like, for acquiring a fluorescence image of the lesion C and a second image-acquisition device 136 for acquiring a visible-light image of the lesion C are provided in the tip portion 32.

The cap 241 is mainly formed of a main body 42, a filter portion 243, an elongated light guide 44, an elongated endoscope channel 45, and a light guiding portion 144 for guiding visible light from the lesion C to the second image-acquisition device 136. When the cap 241 is attached to the tip portion 32, the filter portion 243, the light guiding portion 144, the elongated light guide 44, and the elongated endoscope channel 45 are disposed so as to oppose the first image-acquisition device 135, the second image-acquisition device 136, the light guide 33, and the endoscope channel 34, respectively.

The filter 243 is formed of a lens 47 for guiding light from the lesion C to a splitter 248, the splitter (branching unit) 248 for dividing the incident light into two parts, a first lens 49a and a second lens 49b for focusing the light that is split into two parts onto the first image-acquisition device 135, and a first filter 50a and a second filter 50b which each transmit fluorescence of predetermined wavelengths.

The splitter 248 is provided with a half-mirror 249 which reflects substantially half of the incident light and transmits the rest and a mirror 250 which reflects all of the incident light. The half mirror 249 is disposed so as to maintain an angle of substantially 45° with respect to the light incident on the splitter 248, and the mirror 250 is disposed so that it reflects the light reflected at the half-mirror 249 towards the first image-acquisition device 135.

Next, the operation of the endoscope apparatus 210 having the above-described configuration will be described.

As shown in Fig. 9, in the endoscope apparatus 210, visible light from the lesion C is focused onto a light-receiving surface 136a of the second image-acquisition device 136 by the first light-guiding lens 145 and the second light-guiding lens 146, and a visible-light image is formed. The visible-light image is converted to an electrical signal by the second image-acquisition device 136 and is output to the camera control unit described above.

The light from the lesion C is incident on the splitter 248 via the lens 47. The light incident on the splitter 248 is incident on the half-mirror 249, and substantially half thereof is reflected towards the mirror 250 by the half-mirror 249. The light incident on the mirror 250 is reflected towards the first image-acquisition device 135. The rest of the light transmitted through the half-mirror 249 is emitted from the splitter 235 towards the first image-acquisition device 135.

Of the light incident on the first filter 50a and the second filter 50b via the first lens 49a and the second lens 49b, only the first fluorescence FL1 and the second fluorescence FL2 are transmitted through the filters 50a and 50b, respectively, and form an image on the first image-acquisition device 135. The images of the first fluorescence FL1 and the second fluorescence FL2 are converted to electrical signal by the first image-acquisition device 135 and are output to the camera control unit described above.

As in the second embodiment, the acquired images of each type of light are converted to video signals in the camera control unit, are subjected to signal processing in the image processor, and are thereafter output on the monitor.

A visible-light image P3 of the lesion C is displayed on the left half of the screen on the monitor 65, and a first fluorescence image P1 and a second fluorescence image P2, which have been subjected to signal processing, are displayed on the right half, one above another (see Fig. 8).

According to the configuration described above, using the splitter 248, it is possible to split the light incident on the filter portion 243 into two parts at the half-mirror 249 without degrading the image of that light (the light intensity is substantially halved). In addition, because the split light beams are incident on respective halves of the first image-acquisition device 135, it is possible to reduce the diameter of the tip 32 of the endoscope 31.

Accordingly, the endoscope apparatus 210 can observe images of the two fluorescences FL1 and FL2 at the same time, and it is also possible to reduce the diameter of the tip 32.

The splitter 248 has been described in terms of a splitter in which the ratio (splitting ratio) of the intensity of incident light is 1-to-1, but it is not limited thereto; it is also possible to use a splitter having another splitting ratio.

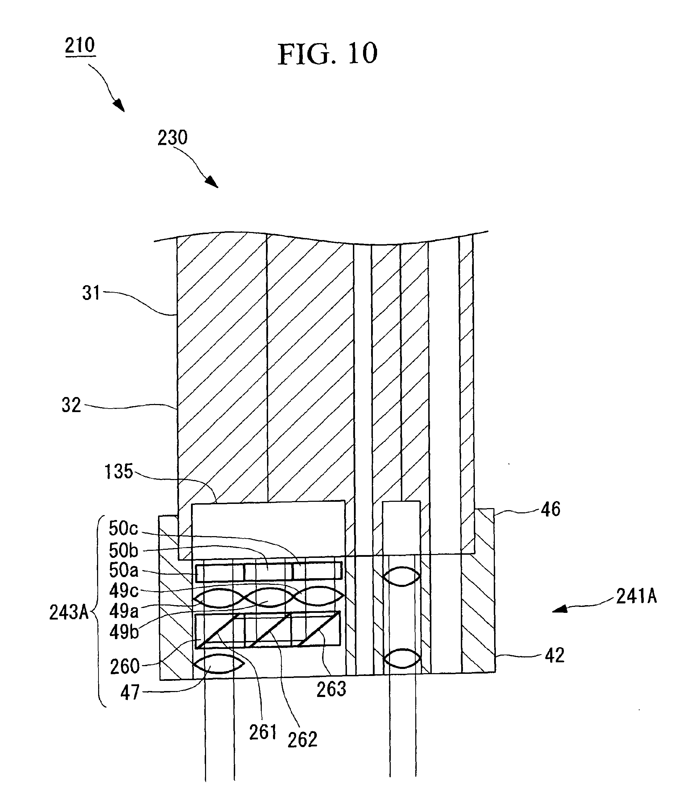

As described above, the endoscope apparatus may use a splitter which divides the incident light into two, or as shown in Fig. 10, it may use a splitter 260 which divides the incident light into three.

More specifically, as shown in Fig. 10, a filter portion 243A of a cap (removable filter apparatus) 241A is formed of a lens 47 for guiding light from the lesion C to the splitter 260; the splitter (branching unit) 260 which divides the incident light into three; a first lens 49a, a second lens 49b, and a third lens 49c which focus the light that is split into three onto the first image-acquisition device 135; and a first filter 50a, a second filter 50b, and a third filter (filter) 50c which respectively transmit fluorescence of predetermine wavelengths.

The splitter 260 is provided with a half-mirror 261 which reflects substantially 1/3 of the incident light and transmits the remaining 2/3; a half-mirror 262 which reflects substantially half of the incident light and transmits the rest; and a mirror 263 which reflects all of the incident light. The half-mirror 261 is disposed so as to maintain and angle of approximately 45° with respect to the light incident on the splitter 260. The half-mirror 262 is disposed so as to reflect the light reflected at the half-mirror 261 towards the first image-acquisition device 135. The mirror 263 is disposed so as to reflect the light transmitted through the half-mirror 262 towards the first image-acquisition device 135.

According to this configuration, using the splitter 260, it is possible to split the light incident on the splitter portion 243A into two parts at the half-mirrors 261 and 262 without degrading the image of that light (the light intensity becomes substantially one-third). In addition, because the split light beams are respectively incident on substantially one-third of the surface of the first image-acquisition device 135, it is possible to reduce the diameter of the tip 32 of the endoscope 31. Therefore, the endoscope apparatus 210 can observe three fluorescence images simultaneously, and it is also possible to reduce the diameter of the tip 32.

The half-mirrors described above may be dichroic prisms.

### Fourth Embodiment

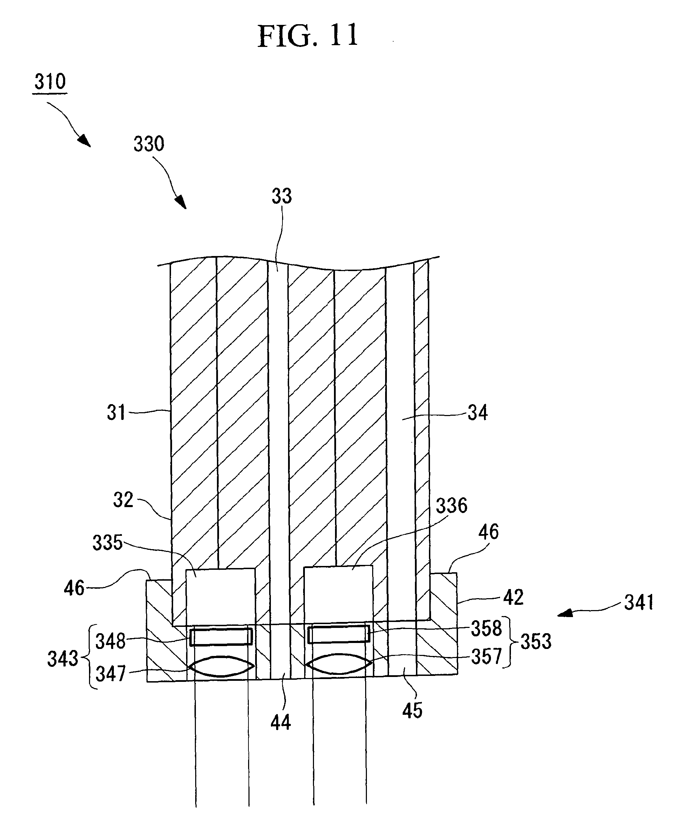

Next, a fourth embodiment of the present invention will be described with reference to Fig. 11.

The basic configuration of the endoscope apparatus according to this embodiment is the same as that of the first embodiment, but the configuration of the endoscope is different from that of the first embodiment. Therefore, in this embodiment, only the vicinity of the endoscope will be described using Fig. 11, and a description of the light-source apparatus and so will be omitted.

Fig. 11 is an enlarged cross-sectional view of a tip portion, which is inserted into a body cavity, of the endoscope apparatus according to this embodiment.

Elements which are identical to those of the first embodiment are assigned the same reference numerals, and a description thereof shall be omitted.

As shown in Fig. 11, an endoscope 330 of an endoscope apparatus 310 is mainly formed of a scope main body 31 and a cap (removable filter apparatus) 341 which is attached to a tip portion 32 thereof in such a manner that it can be attached thereto and removed therefrom.

The tip portion 32 is provided with a first image-acquisition device (solid-state image-acquisition device) 335, such as a CCD device, for acquiring a visible-light image and a fluorescence image of the lesion C and a second image-acquisition device (solid-state image-acquisition device) 336 for acquiring a fluorescence image of the lesion C.

The cap 341 is mainly formed of a main body 42, a first filter portion 343, a second filter portion 353, an elongated light guide 44, and an elongated endoscope channel 45. When the cap 341 is attached to the tip portion 32, the first filter portion 343, the second filter portion 353, the elongated light guide 44, and the elongated endoscope channel 45 are disposed so as to oppose the first image-acquisition device 335, the second image-acquisition device 336, the light guide 33, and the endoscope channel 34, respectively.

The first filter portion 343 is formed of a first lens 347 for guiding light from the lesion C to a first filter (filter) 348, and the first filter 348 for transmitting visible light and first fluorescence FL1 among the light incident thereon.

The second filter portion 353 is formed of a second lens 357 for guiding light from the lesion C to a second filter (filter) 358, and the second filter 358 for transmitting second fluorescence FL2 among the light incident thereon.

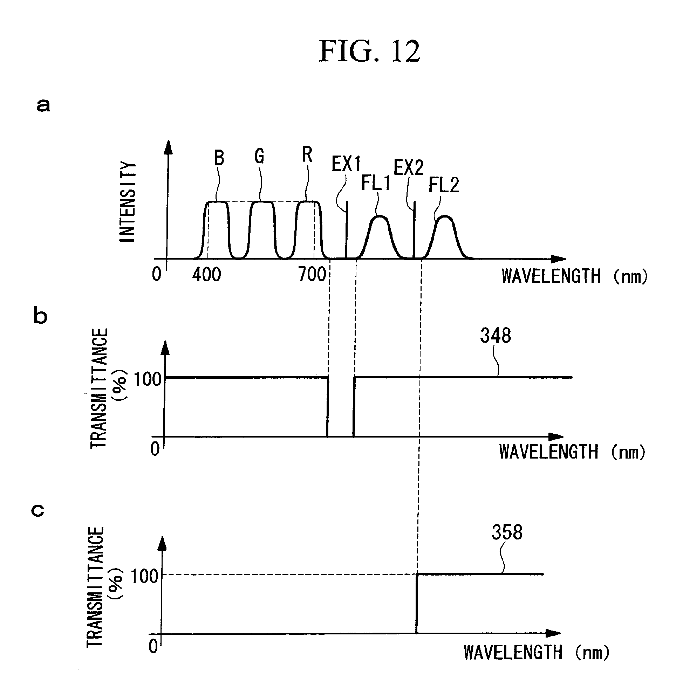

Fig. 12(a) is a diagram showing the relationship of the wavelengths of the visible light and the wavelengths of each of the light beams EX1, EX2, FL1, and FL2. Fig. 12(b) is a diagram showing the light transmittance characteristic of the first filter 348 according to this embodiment, and Fig. 12(c) is a diagram showing the light transmittance characteristic of the second filter 358 according to this embodiment.

As shown in Fig. 12(c), a filter exhibiting a transmittance characteristic for visible light and the first fluorescence FL1 and exhibiting a blocking characteristic for the first excitation light EX1 is used as the first filter 348. As shown in Fig. 12(d), a filter exhibiting a transmittance characteristic for the second fluorescence FL2 and light with a longer wavelength and exhibiting a blocking characteristic for light with a shorter wavelength than the second fluorescence is used as the second filter 358.

Next, the operation of the endoscope apparatus 310 having the above-described configuration will be described.

As shown in Fig. 11, in the endoscope apparatus 310, light from the lesion C is incident on the first lens 347 and the second lens 357 and is focused so as to form an image on the first image-acquisition device 335 and the second image-acquisition device 336, respectively. The light beams transmitted through the first lens 347 and the second lens 357 are incident on the first filter 348 and the second filter 358, respectively.

As shown in Fig. 12(b), in the first filter 348, only the first excitation light EX1 is blocked, and the other light which is transmitted through the first filter 348 forms an image on the first image-acquisition device 335. As shown in Fig. 12(c), in the second filter 358, light with a shorter wavelength than the second fluorescence FL2 is blocked, and the second fluorescence FL2 which is transmitted through the second filter 358 forms an image on the second image-acquisition device 336.

The first image-acquisition device 335 and the second image-acquisition device 336 convert the formed images into electrical signals and output them to the camera control unit described above.

Images of the second excitation light EX2 and the second fluorescence FL2 are also formed on the first image-acquisition device 335, but image acquisition of the images of the second excitation light EX2 and the second fluorescence FL2 is not performed in the first image-acquisition device 335.

As in the first embodiment, the acquired images of each light beam are converted to video signals in the camera control unit, are subjected to signal processing in the image processor, and are thereafter output onto the monitor.

A visible-light image P3 of the lesion C is displayed on the left half of the screen on the monitor 65, and a first fluorescence image P1 and a second fluorescence image P2, which are subjected to signal processing, are displayed on the right half of the screen, one above the other (see Fig. 8).

According to this configuration, it is possible to make the first fluorescence FL1 and the second fluorescence FL2, which are transmitted through the first filter 347 and the second filter 358, respectively, incident on the first image-acquisition device 335 and the second image-acquisition device 336. Accordingly, the first image-acquisition device 335 and the second image-acquisition device 336 can respectively detect the first fluorescence FL1 and the second fluorescence FL2 from the lesion C.

Because the first filter 347 transmits visible light (light including blue light B, green light G, and red light R), it is possible to observe a visible-light image at the lesion C. Therefore, it is possible to simultaneously observe the visible-light image P3 and the fluorescence images P1 and P2, and by using these images, it is possible to more accurately identify the position of cells involved in the pathology.

The technical scope of the present invention is not limited to the embodiments described above; various modifications can be applied so long as they do not depart from the spirit of the present invention.

For example, although the above embodiments have been described in terms of a configuration in which one or two solid-state image-acquisition devices are provided, the number of image-acquisition devices is not limited to one or two; it is also possible to use a configuration in which three or more are provided.

## Claims

1. A removable filter apparatus which is attached, in an attachable and removable manner, to a tip of an inserting portion of an endoscope apparatus including a projection unit for emitting light towards biological tissue and a solid-state image-acquisition device for detecting return light from the biological tissue, the removable filter apparatus comprising:
a plurality of filters corresponding to fluorescences of different wavelengths in the return light from the biological tissue,
wherein the plurality of filters block light with wavelengths shorter than the wavelengths of the corresponding fluorescences.

2. A removable filter apparatus according to claim 1, wherein the plurality of filters block light with wavelengths longer than the wavelengths of the corresponding fluorescences.

3. A removable filter apparatus according to Claim 1 or Claim 2, wherein the plurality of filters are disposed at positions which cover parts of light-receiving surfaces of the corresponding solid-state image-acquisition devices; and
a branching unit for branching the return light from the biological tissue towards the plurality of filters is provided.

4. A removable filter apparatus according to Claim 3, wherein the branching unit is a polarizer.

5. A removable filter apparatus according to Claim 3, wherein the branching unit is a splitter.

6. A removable filter apparatus according to one of Claims 1 to 5, wherein two of the solid-state image-acquisition devices are provided; and
at least one of the filters is disposed at a position which covers the light-receiving surfaces of the respective solid-state image-acquisition devices.

7. A removable filter apparatus according to one of Claims 1 to 6, wherein, of the plurality of filters, at least one of the filters transmits visible light.

8. A removable filter apparatus according to one of Claims 1 to 7, further comprising an elongated light guide for guiding the light emitted from the projection unit to an end surface.

9. A removable filter apparatus according to one of Claims 1 to 8, wherein an elongated treatment-instrument channel, which extends a treatment-instrument channel provided in the endoscope apparatus to an end surface, is formed.

10. An endoscope apparatus comprising:
a light source for emitting light;
a switching mechanism for converting the light emitted from the light source into light of a plurality of different wavelengths;
a light guide, disposed inside an inserting portion for inserting into a body cavity, for guiding the light converted by the switching mechanism to a tip of the inserting portion and for emitting the light towards biological tissue;
a solid-state image-acquisition device, disposed at the tip of the inserting portion, for detecting return light from the biological tissue; and
a removable filter apparatus which is attached, in an attachable and removable manner, to the tip of the inserting portion, and which includes a plurality of filters for transmitting fluorescences of different wavelengths among the return light from the biological tissue and for blocking excitation light which generates the fluorescences.
